# EUROPEAN PATENT APPLICATION

(11) **EP 4 775 603 A1**
(43) Date of publication of application: **15.07.2026**
(21) Application number: 24862503.0
(22) Date of filing: 07.08.2024
(51) Int. Cl.: C08F 20/14, C08F 2/44

(54) **COMPOSITION, POLYMER, CURED PRODUCT, MOLDED BODY, AND METHOD FOR PRODUCING POLYMETHYL METHACRYLATE**

(30) Priority: 04.09.2023 JP 2023143082
(71) Applicant: Sumitomo Chemical Company, Limited, Tokyo 103-6020 (JP)
(72) Inventor: YASUTOMI, Yoichi, Niihama-shi, Ehime 792-8521 (JP); KADOYA, Hidenori, Niihama-shi, Ehime 792-8521 (JP)
(74) Representative: Vossius & Partner Patentanwälte Rechtsanwälte mbB
(86) International application number: PCT/JP2024/028236
(87) International publication number: WO 2025/052849

(57) **Abstract**

An object of the present invention is to provide a composition having excellent storage stability, a polymer, a cured product, and a molded body obtained using the composition, and a method for producing polymethyl methacrylate using the composition. The present invention relates to a composition containing methyl methacrylate and toluene, in which a concentration of toluene is greater than 0 mass ppm and less than 1,000 mass ppm based on the total composition.

## Description

### TECHNICAL FIELD

The present disclosure relates to a composition, a polymer, a cured product, a molded body, and a method for producing polymethyl methacrylate.

### BACKGROUND ART

Poly(methyl (meth)acrylate) obtained by polymerizing methyl (meth)acrylate is used in various fields as a resin material having excellent transparency and weather resistance. As resource prices have risen in recent years and awareness of environmental problems has grown, products (molded bodies) containing poly(methyl (meth)acrylate) used for various applications as described above have been recovered and recycled.

Examples of a method for recycling poly(methyl (meth)acrylate) include material recycling in which a recovered molded body is subjected to a molding step again to produce a new molded body, chemical recycling in which methyl (meth)acrylate is recovered by subjecting a recovered molded body to heat treatment to thermally decompose (depolymerize) poly(methyl (meth)acrylate), and a new molded body is produced using the recovered methyl (meth)acrylate (may be referred to as recycled MMA or recycled MA), and thermal recycling in which a recovered molded body is incinerated as fuel, the combustion energy is directly used as a heat source, and furthermore, the combustion energy is utilized for power generation.

In addition, in response to diversification of applications of poly(methyl (meth)acrylate) in recent years, a technique for improving the quality of poly(methyl (meth)acrylate) has been studied.

For example, as a polymerization apparatus suitable for obtaining high-quality poly(methyl (meth)acrylate), a polymerization apparatus in which formation of a gel in a reaction vessel for reacting a raw material monomer with a polymerization initiator is suppressed has been proposed (see Patent Document 1).

### PRIOR ART DOCUMENT

### PATENT DOCUMENT

Patent Document 1: JP-A-2012-102190

### SUMMARY OF THE INVENTION

### PROBLEMS TO BE SOLVED BY THE INVENTION

In addition to the improvement in the polymerization process of the raw material monomer as described in Patent Document 1, measures for suppressing deterioration in the quality of the raw material monomer during storage can be mentioned as measures for improving the quality of poly(methyl (meth)acrylate).

In view of the above circumstances, an object of an embodiment of the present disclosure is to provide a composition having excellent storage stability, particularly excellent storage stability when stored for a long period of time, a polymer, a cured product, and a molded body obtained using the composition, and a method for producing polymethyl methacrylate using the composition.

### MEANS FOR SOLVING THE PROBLEMS

Means for solving the above problems includes the following embodiments.
<1> A composition containing methyl methacrylate and toluene, in which a concentration of toluene is greater than 0 mass ppm and less than 1,000 mass ppm based on the total composition.
<2> The composition according to <1>, in which a content of methyl methacrylate is 85 mass% or more based on the total composition.
<3> The composition according to <1> or <2>, in which a content of methyl methacrylate is 90 mass% or more based on the total composition.
<4> The composition according to any one of <1> to <3>, in which the concentration of toluene is 5 mass ppm to 700 mass ppm based on the total composition.
<5> The composition according to any one of <1> to <4>, in which the concentration of toluene is 100 mass ppm to 600 mass ppm based on the total composition.
<6> The composition according to any one of <1> to <5>, in which the methyl methacrylate includes recycled methyl methacrylate or bio-derived methyl methacrylate.
<7> The composition according to any one of <1> to <6>, further containing a (meth)acrylic acid ester other than methyl methacrylate.
<8> The composition according to any one of <1> to <7>, further containing a polymer containing a structural unit derived from methyl methacrylate.
<9> A polymer containing a structural unit derived from the methyl methacrylate contained in the composition according to any one of <1> to <8>.
<10> A molded body containing the polymer according to <9>.
<11> A cured product of the composition according to any one of <1> to <8>.
<12> A molded body including the cured product according to <11>.
<13> A method for producing polymethyl methacrylate, the method including polymerizing the methyl methacrylate contained in the composition according to any one of <1> to <8>.

### EFFECT OF THE INVENTION

According to an embodiment of the present disclosure, there are provided a composition having excellent storage stability, particularly excellent storage stability when stored for a long period of time, a polymer, a cured product, and a molded body obtained using the composition, and a method for producing polymethyl methacrylate using the composition.

### BRIEF DESCRIPTION OF THE DRAWING

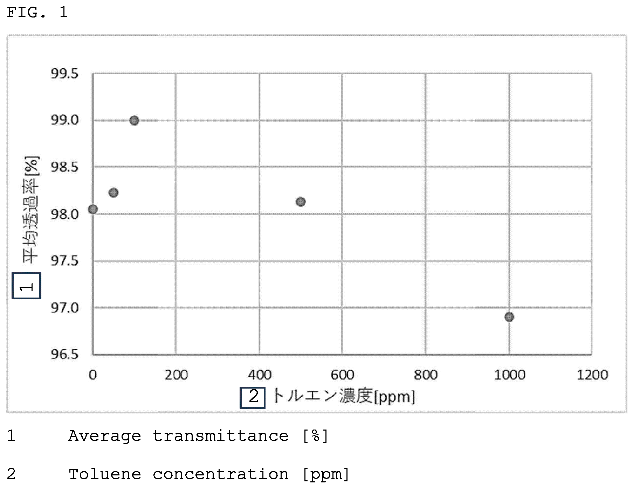

Fig. 1 is a graph showing the relationship between the concentration of toluene and the average transmittance in the compositions prepared in Examples.

### MODE FOR CARRYING OUT THE INVENTION

Hereinafter, embodiments of the present invention will be described. However, the present invention is not limited to the following embodiments.

In the present specification, a numerical range indicated using "to" includes numerical values before and after "to" as the minimum value and the maximum value, respectively.

In the numerical ranges described in stages in the present specification, an upper limit value or a lower limit value described in one numerical range may be replaced with an upper limit value or a lower limit value of the numerical range described in another stage. In addition, in the numerical range described in the present specification, the upper limit value or the lower limit value of the numerical range may be replaced with a value shown in Examples.

### <Composition>

A composition of the present disclosure is a composition containing methyl methacrylate and toluene, in which a concentration of toluene is greater than 0 mass ppm and less than 1,000 mass ppm based on the total composition. The composition of the present disclosure may be in a liquid form or in a solid form such as pellets. In the case of a liquid form, the composition may be, for example, a monomer composition, or may contain a polymer of the monomer, like a syrup.

As shown in Examples described below, the composition of the present disclosure has excellent storage stability.

### (Methyl Methacrylate)

The composition of the present disclosure contains methyl methacrylate.

In the present disclosure, "methyl methacrylate" refers to methyl methacrylate that is substantially free of impurities such as by-products generated during synthesis of methyl methacrylate. However, methyl methacrylate in the present disclosure is not limited thereto, as long as the object of the invention is not impaired. In other words, "methyl methacrylate" may contain impurities that cannot be completely removed by an ordinary purification method or may contain impurities in a content that cannot be detected by an ordinary detection method.

A content of methyl methacrylate in the composition is not particularly limited, and can be selected according to the application of polymethyl methacrylate obtained using the composition, and the like. The content of the methyl methacrylate may be, for example, 85 mass% or more, 90 mass% or more, 95 mass% or more, or 99 mass% or more based on the total composition.

When the content of methyl methacrylate in the composition is within the above range, a polymer obtained by polymerizing the composition and a molded body containing the same are preferable from the viewpoint of at least heat resistance or transparency (light transmittance) thereof.

The methyl methacrylate contained in the composition may be synthesized by a known synthesis method. The synthesis method is not particularly limited, and may be an ACH method, a C4 direct oxidation method, or an alpha method.

The methyl methacrylate contained in the composition may include recycled methyl methacrylate.

In the present disclosure, recycled methyl methacrylate refers to methyl methacrylate obtained by depolymerization of polymethyl methacrylate (reaction in which a polymer is decomposed to produce a monomer).

The depolymerization of polymethyl methacrylate can be performed, for example, by heat treatment of polymethyl methacrylate.

A supply source of polymethyl methacrylate as a raw material of recycled methyl methacrylate is not particularly limited as long as methyl methacrylate can be recovered. For example, the supply source of polymethyl methacrylate may be a molded body containing polymethyl methacrylate.

The methyl methacrylate contained in the composition may include bio-derived methyl methacrylate.

In the present disclosure, the bio-derived methyl methacrylate refers to methyl methacrylate synthesized from a bio-derived raw material. The bio-derived raw material may be a plant-derived raw material or an animal-derived raw material, and is preferably a plant-derived raw material.

### (Toluene)

The composition of the present disclosure contains toluene.

A concentration of toluene contained in the composition is greater than 0 mass ppm and less than 1,000 mass ppm based on the total composition.

From the viewpoint of long-term storage stability of the composition, the concentration of toluene contained in the composition is preferably 5 mass ppm or more, more preferably 50 mass ppm or more, and still more preferably 100 mass ppm or more based on the total composition.

From the viewpoint of optical stability of the composition, the concentration of toluene contained in the composition is preferably 700 mass ppm or less, more preferably 600 mass ppm or less, still more preferably 500 mass ppm or less, particularly preferably 400 mass ppm or less, and particularly preferably 300 mass ppm or less based on the total composition.

If necessary, the composition may contain a component that does not correspond to methyl methacrylate and toluene. For example, the composition may contain a (meth)acrylic acid ester other than methyl methacrylate described below, a polymer containing a structural unit derived from methyl methacrylate, a low-content component, or an additive.

### ((Meth)acrylic Acid Ester)

The composition may contain, in addition to methyl methacrylate, a (meth)acrylic acid ester other than methyl methacrylate (hereinafter, also simply referred to as (meth)acrylic acid ester).

Specific examples of the (meth)acrylic acid ester include methyl acrylate, ethyl (meth)acrylate, propyl (meth)acrylate, n-butyl (meth)acrylate, sec-butyl (meth)acrylate, tert-butyl (meth)acrylate, cyclohexyl (meth)acrylate, benzyl (meth)acrylate, 2-ethylhexyl (meth)acrylate, 2-hydroxyethyl (meth)acrylate, and cyclopentanyl (meth)acrylate. Among them, methyl acrylate or ethyl acrylate is preferable, and methyl acrylate is more preferable. These (meth)acrylic acid esters may be used alone or as a mixture of two or more kinds thereof.

In the present disclosure, the "(meth)acrylic acid ester" refers to an acrylic acid ester or a methacrylic acid ester.

The (meth)acrylic acid ester may be contained in the composition as a by-product generated during the production of methyl methacrylate or the recycling treatment of polymethyl methacrylate, or may be contained in the composition after being intentionally mixed.

When the composition contains a (meth)acrylic acid ester, a concentration thereof is preferably 50,000 ppm by mass or less, more preferably 40,000 ppm by mass or less, and still more preferably 30,000 ppm by mass or less based on the total composition.

When the composition contains another (meth)acrylic acid ester, a concentration thereof may be 1 ppm by mass or more, 2 ppm by mass or more, or 5 ppm by mass or more based on the total composition.

The composition may contain a polymer containing a structural unit derived from methyl methacrylate in addition to methyl methacrylate. The polymer may contain a structural unit derived from methyl methacrylate, and may be a homopolymer of methyl methacrylate or a copolymer of methyl methacrylate and another (meth)acrylic acid ester polymerizable with methyl methacrylate. Examples of the other (meth)acrylic acid ester polymerizable with methyl methacrylate include the same as those described above.

### (Low-Content Component)

The composition may contain a low-content component other than toluene. The low-content component may be contained in the composition as a by-product generated during the production of methyl methacrylate or the recycling treatment of polymethyl methacrylate.

In the present disclosure, the low-content component refers to a component contained in the composition at a concentration of less than 1,000 mass ppm.

Examples of the low-content component other than toluene that may be contained in the composition include a carboxylic acid ester, an aromatic hydrocarbon compound, an aliphatic hydrocarbon compound, an alcohol, and butyl acrylate.

The low-content component other than toluene contained in the composition may be only one kind or two or more kinds.

Examples of the carboxylic acid ester include methyl isobutyrate, methyl propionate, methyl 2,4-dimethyl-4-pentenoate, methyl 2-methyl-3-butenoate, methyl tiglate, methyl 3-methyl-3-butenoate, methyl 3-methyl-2-butenoate, dimethyl itaconate, and dimethyl 2-methyl-5-methylenehexanedioate.

Specific examples of the aromatic hydrocarbon compound include styrene.

Specific examples of the aliphatic hydrocarbon compound include 1-octene and 1-octadecene.

When the composition contains low-content components other than toluene, the concentration of each low-content component is preferably 700 mass ppm or less based on the total composition. In addition, the concentration of each low-content component may be 1 mass ppm or more, 2 mass ppm or more, or 5 mass ppm or more based on the total composition.

### (Additive)

If necessary, the composition may contain an additive. Specific examples of the additive include a release agent, a polymerization regulator, a polymerization initiator, an ultraviolet absorber, and a colorant.

The additive contained in the composition may be only one kind or two or more kinds.

Examples of the release agent that may be contained in the composition include a higher fatty acid ester, a higher aliphatic alcohol, a higher fatty acid, a higher fatty acid amide, a higher fatty acid metal salt, and a fatty acid derivative.

Specific examples of the release agent that may be contained in the composition include sodium di(2-ethylhexyl) sulfosuccinate, stearyl alcohol, methyl stearate, and stearamide.

The release agent contained in the composition may be only one kind or two or more kinds.

A content of the release agent in the composition can be, for example, 0.01 mass% to 1.0 mass% based on the total composition.

As the polymerization regulator (an additive for adjusting a polymerization rate in a polymerization reaction) that may be contained in the composition, any suitable conventionally known polymerization regulator can be used. Examples of the polymerization regulator include compounds that can control a polymerization rate so as to reduce the polymerization rate.

Specific examples of the polymerization regulator include mercaptan compounds such as n-butyl mercaptan and n-octyl mercaptan; terpenoid compounds such as limonene, myrcene, α-terpinene, β-terpinene, γ-terpinene, terpinolene, β-pinene, and α-pinene; and an α-methylstyrene dimer.

The polymerization regulator contained in the composition may be only one kind or two or more kinds.

A content of the polymerization regulator in the composition can be, for example, 0.001 mass% to 0.5 mass% based on the total composition.

Examples of the polymerization initiator that may be contained in the composition include a radical polymerization initiator, a diacyl peroxide initiator, a dialkyl peroxide initiator, a peroxyester initiator, a percarbonate initiator, and a peroxyketal initiator.

Specific examples of the radical polymerization initiator include azo compounds such as 1,1'-azobis(cyclohexane-1-carbonitrile), 2,2'-azobis(2,4,4-trimethylpentene), 2,2'-azobis(2-methylpropane), 2-cyano-2-propylazoformamide, 2,2'-azobis(2-hydroxy-methylpropionate), 2,2'-azobis(2-methyl-butyronitrile), 2,2'-azobisisobutyronitrile, 2,2'-azobis[2-(2-imidazolin-2-yl)propane], and dimethyl 2,2'-azobis(2-methylpropionate).

Specific examples of the diacyl peroxide initiator and the dialkyl peroxide initiator include dicumyl peroxide, tert-butyl cumyl peroxide, di-tert-butyl peroxide, benzoyl peroxide, and lauroyl peroxide.

Specific examples of the peroxyester initiator include tert-butyl peroxy-3,3,5-trimethylhexanoate, tert-butyl peroxylaurate, tert-butyl peroxyisobutyrate, tert-butyl peroxyacetate, di-tert-butyl peroxyhexahydroterephthalate, di-tert-butyl peroxyazelate, tert-butyl peroxy-2-ethylhexanoate, 1,1,3,3-tetramethylbutyl peroxy-2-ethylhexanoate, and tert-amyl peroxy-2-ethylhexanoate.

Specific examples of the percarbonate initiator include tert-butyl peroxyallyl carbonate and tert-butyl peroxyisopropyl carbonate.

Specific examples of the peroxyketal initiator include 1,1-di-tert-butylperoxycyclohexane, 1,1-di-tert-butylperoxy-3,3,5-trimethylcyclohexane, and 1,1-di-tert-hexylperoxy-3,3,5-trimethylcyclohexane.

The polymerization initiator contained in the composition may be only one kind or two or more kinds.

A content of the polymerization initiator in the composition can be, for example, 0.01 mass% to 5 mass% based on the total composition.

Examples of the ultraviolet absorber that may be contained in the composition include a benzophenone ultraviolet absorber, a cyanoacrylate ultraviolet absorber, a benzotriazole ultraviolet absorber, a malonic acid ester ultraviolet absorber, and an oxalanilide ultraviolet absorber.

Specific examples of the ultraviolet absorber include 2-(2'-hydroxy-5'-methylphenyl)benzotriazole, 2-hydroxy-4-n-octylbenzophenone, 2-(3-tert-butyl-2-hydroxy-5-methylphenyl)-5-chlorobenzotriazole, 2-(2-hydroxy-3,5-di-tert-pentylphenyl)benzotriazole, and 2,4-di-tert-butylphenyl-3,5-di-tert-butyl-4-hydroxybenzoate.

The ultraviolet absorber contained in the composition may be only one kind or two or more kinds.

A content of the ultraviolet absorber in the composition can be, for example, 0.001 mass% to 1 mass% based on the total composition.

Examples of the colorant that may be contained in the composition include a perylene dye, a perinone dye, a pyrazolone dye, a methine dye, a coumarin dye, a quinophthalone dye, a quinoline dye, an anthraquinone dye, an asdrapyridone dye, a thioindigo dye, a coumarin dye, an isoindolinone pigment, a diketopyrrolopyrrole pigment, a condensed azo pigment, a benzimidazolone pigment, a dioxazine pigment, a copper phthalocyanine pigment, and a quinacridone pigment.

The colorant contained in the composition may be only one kind or two or more kinds.

A content of the colorant in the composition can be, for example, 1.0 × 10⁻⁸ mass% to 0.5 mass% based on the total composition.

When the composition contains an additive, the total content thereof may be 15 mass% or less, 10 mass% or less, 5 mass% or less, or 1 mass% or less based on the total composition.

When the composition contains an additive, the total content thereof may be 0.01 mass% or more, 0.05 mass% or more, or 0.1 mass% or more based on the total composition.

The composition of the present disclosure may be used immediately after preparation, or may be used after storage. The storage conditions for storing the composition are not particularly limited, and can be, for example, 0°C to 45°C. From the viewpoint of ensuring excellent quality, the temperature during storage is preferably selected from a range of 0°C to 39°C, more preferably from a range of about 25°C ± 10°C, and still more preferably from a range of about 25 to 30°C.

The composition of the present disclosure has excellent storage stability. Specifically, the transmittance of the composition after storage is improved as compared with the case where toluene is not added. Accordingly, the composition of the present disclosure can maintain high transmittance even when stored for a long period of time. In the present disclosure, the storage stability of the composition was determined by measuring the transmittance of the composition stored under accelerated conditions. Hereinafter, a method for measuring transmittance, which was adopted for the evaluation of storage stability, will be described.

In Examples described below, the evaluation of the storage stability of the composition is performed in the following order: (1) preparation of a composition containing methyl methacrylate and toluene; (2) a storage test including the following Steps 1 to 7 in this order; and (3) measurement of the transmittance of the composition stored under accelerated conditions.

Step 1: Inject 25 mL of the composition into a lower part of a pressure vessel ("TVS-N2 type" manufactured by Taiatsu Techno Corporation).

Step 2: A packing was interposed between the upper part and the lower part of the pressure vessel, and the pressure vessel was sealed.

Step 3: Nitrogen was introduced from the tip of the upper part of the pressure vessel, and sealed in a state where the internal pressure was 0.2 MPa, and it was confirmed whether the internal pressure did not change for 1 minute.

Step 4: The internal pressure in the pressure vessel was released, and a closing plug was attached to the tip of the upper part of the pressure vessel.

Step 5: The pressure vessel is placed in an oil bath set at 80°C.

Step 6: The pressure vessel is stored in the oil bath for 24 hours.

Step 7: After 24 hours had elapsed, the pressure vessel was taken out of the oil bath and placed in ice-cooled water for quenching.

In Examples described below, the transmittance of the composition stored under the accelerated conditions is measured by measuring the transmittance of the composition after storage. Specifically, using the composition after storage, the average transmittance (%) is measured for light having a wavelength in a range of 300 nm to 370 nm when an optical path length is 10 mm. The average transmittance refers to an average value of transmittance values measured at every 5 nm in the wavelength range of 300 nm to 370 nm. For the measurement, a spectrophotometer ("Hitachi Spectrophotometer U-4000", manufactured by Hitachi High-Tech Fielding Corporation) is used; however, any suitable commercially available spectrophotometer may be adopted.

An increase in the transmittance of the composition indicates an improvement in transparency. In addition, as the transmittance after storage increases, it can be determined that the optical properties of the composition, such as transparency, are maintained.

The average transmittance of the composition stored under the accelerated conditions is preferably 98.10% or more, and more preferably 98.50% or more. A composition containing methyl methacrylate is often used for a molded body requiring transparency, and a composition exhibiting such transmittance is useful for a molded body requiring transparency.

In the composition of the present disclosure, by mixing toluene and methyl methacrylate at a predetermined ratio, the transmittance after storage is improved as compared with a composition not containing toluene, and high transmittance is exhibited even after storage. This effect can be said to be an unexpected and remarkable effect. Although the reason for this is not clear, it is considered that the addition of a small amount of toluene to methyl methacrylate causes some kind of action, such as an interaction between monomers, during storage.

### <Polymer, Cured Product, and Molded Body>

A polymer of the present disclosure is a polymer containing a structural unit derived from the methyl methacrylate contained in the composition of the present disclosure described above. Here, the polymer is obtained by polymerizing a component contained in the composition that is involved in polymerization, and contains a structural unit derived from the component involved in polymerization. The polymer of the present disclosure may contain a structural unit derived from methyl methacrylate contained in the composition of the present disclosure and a structural unit derived from another polymerization component. A weight average molecular weight of the polymer of the present disclosure is not particularly limited, and can be selected according to the application of the polymer.

A cured product of the present disclosure is a cured product of the composition of the present disclosure described above. Here, the cured product is obtained by curing the composition, and may contain a component not involved in polymerization. However, depending on the method for curing the composition, it can also be considered the same as the polymer described above (that is, containing no component that is not involved in polymerization).

A molded body of the present disclosure contains the polymer or cured product of the present disclosure described above. When the cured product can be regarded as identical to the polymer, the molded body contains the polymer of the composition of the present disclosure. The molded body is preferably a molded body containing a cured product obtained by curing only the composition of the present disclosure. The molded body may be an article formed by molding a polymer or a cured product into an arbitrary shape.

### (Physical Properties of Polymer and Cured Product)

The polymer and the cured product of the present disclosure have excellent optical properties. Specifically, the polymer or the cured product of the present disclosure tends to exhibit superior optical properties, such as transparency, compared with a polymer or a cured product obtained from a composition that contains methyl methacrylate and does not contain toluene. Polymers or cured products of methyl methacrylate compositions are often required to exhibit high transparency for their applications. Therefore, according to the composition of the present application, it is possible to provide a polymer or a cured product having higher transparency than a polymer or a cured product of a composition not containing toluene. In addition, the composition of the present application exhibits higher transmittance than a composition not containing toluene even after the composition is stored for a long period of time. Therefore, according to the composition of the present application, a polymer or a cured product having high transparency can be provided even after the composition is stored for a long period of time.

The transparency of the polymer and the cured product can be determined by measuring the transmittance. In the present disclosure, the measurement of transmittance may be performed on a produced polymer or a cured product, or may be performed after subjecting the polymer or the cured product to an accelerated deterioration treatment. By this method, the transparency after using the polymer or the cured product for a long time can be efficiently evaluated.

The transmittance of the polymer or the cured product can be measured by, for example, the following method. First, a test piece with a size of 50 mm × 50 mm × 3 mm prepared from the polymer or the cured product is subjected to an accelerated deterioration treatment in which the test piece is allowed to stand in a thermostatic chamber ("Vacuum Oven VOS-301SD", manufactured by TOKYO RIKAKIKAI CO., LTD.) set at 80°C for 200 hours. Next, the average transmittance (%) in light having a wavelength in a range of 380 nm to 780 nm when an optical path length is 50 mm is measured using the test piece after the accelerated deterioration treatment. The measurement is performed using a spectrophotometer ("Hitachi Spectrophotometer U-4000" manufactured by Hitachi High-Tech Fielding Corporation). The average transmittance of the polymer or cured product after the accelerated deterioration treatment is preferably 81% or more and more preferably 81.5% or more from the viewpoint of optical properties.

Whether or not the polymer and the cured product of the present disclosure contain methyl methacrylate, toluene, or other components can be identified by a known analysis method. Examples of the known analysis method include gas chromatography and liquid chromatography.

The polymer, the cured product, and the molded body of the present disclosure contain toluene in an amount of greater than 0 mass ppm and 1,000 mass ppm or less based on the total mass of the polymer, the cured product, and the molded body.

The concentration of toluene contained in the polymer, the cured product, or the molded body is preferably 5 mass ppm or more, more preferably 50 mass ppm or more, and still more preferably 100 mass ppm or more based on the total mass thereof.

The upper limit value of the concentration of toluene contained in the polymer, the cured product, or the molding is not particularly limited, and may be, for example, 800 mass ppm or less, 700 mass ppm or less, 600 mass ppm or less, or 500 mass ppm or less.

Furthermore, when the concentration of toluene is in a range of less than 1,000 mass ppm, the transparency of a molded body obtained from the composition tends to be high.

From the viewpoint of the transparency of the molded body, the concentration of toluene contained in the polymer, the cured product, or the molded body is preferably 800 mass ppm or less, more preferably 700 mass ppm or less, still more preferably 600 mass ppm or less, even more preferably 500 mass ppm or less, and particularly preferably 400 mass ppm or less based on the total mass of the polymer, the cured product, or the molded body.

### <Method for Producing Polymethyl Methacrylate>

The method for producing polymethyl methacrylate of the present disclosure is a method for producing polymethyl methacrylate, the method including polymerizing methyl methacrylate contained in the composition of the present disclosure described above. Here, the polymethyl methacrylate of the present disclosure also corresponds to a polymer obtained from the composition of the present disclosure, and also corresponds to a cured product obtained from the composition.

The method for polymerizing methyl methacrylate contained in the composition is not particularly limited, and the polymerization may be performed by a known method. For example, the polymerization may be performed by a method such as bulk polymerization, cell cast polymerization, solution polymerization, suspension polymerization, or emulsion polymerization.

Specifically, a sheet (molded body) obtained by molding a polymer of methyl methacrylate from the composition of the present disclosure by, for example, a bulk polymerization method can be obtained. In addition, in cell cast polymerization, a cured product (molded body) obtained by curing the composition can be obtained by performing heat treatment under predetermined heating conditions to advance the polymerization reaction.

In the method for polymerizing methyl methacrylate contained in the composition of the present disclosure or the method for producing a molded body, the heating temperature and heating time among the heating conditions can be set, for example, in consideration of the type and content of the selected polymerization regulator, polymerization initiator, and/or other components.

In the cell cast polymerization, the heating temperature can be set to, for example, 50°C to 120°C in producing a cured product and a molded body thereof. In addition, the heating time in the production can be, for example, 1 hour to 20 hours. In addition, the heat treatment may be a heat treatment including a plurality of steps with different heating temperatures and/or heating times.

A cured product obtained by cell cast polymerization and a molded body thereof can be produced by heat treatment under heating conditions including, for example, the following Steps 1 to 7.

Step 1: Raise the temperature from room temperature to 68°C for 20 minutes.

Step 2: The temperature was maintained at 68°C for 90 minutes.

Step 3: The temperature was lowered from 68°C to 64°C for 20 minutes.

Step 4: The temperature was maintained at 64°C for 90 minutes.

Step 5: The temperature was raised from 64°C to 123°C for 10 minutes.

Step 6: Maintain the temperature at 123°C for 120 minutes.

Step 7: Lower the temperature from 123°C to room temperature for 78 minutes.

In the method for producing a cured product and a molded body thereof, Steps 1 to 7 are performed in this order, such that it is possible to suppress heat generation during the polymerization reaction and to stably complete the polymerization.

In the heat treatment of the composition of the present disclosure, for example, a cell cast method (cell cast polymerization) is applied using a cell that defines a predetermined-shaped sealed space inside the cell, thereby forming a molded body with a predetermined shape. Hereinafter, a method for producing a molded body by the cell cast method will be described in detail.

In producing a molded body by the cell cast method, first, a cell is prepared. Here, an example of forming a plate-like molded body (may be referred to as "cast plate") will be described.

Such a cell can include at least two flat-plate members and a seal material (gasket) that is disposed so as to be sandwiched between the two flat-plate members and can seal a gap between the two opposing flat-plate members as a closed space.

The flat-plate member may be in the form of a single sheet or a belt. The flat-plate member is composed of a material that is not dissolved by the composition of the present disclosure, does not inhibit the polymerization reaction of the composition, and has sufficient heat resistance for the heating temperature during the heat treatment. Examples of a preferred material for the flat-plate member include glass and a metal.

As the seal material, any suitable conventionally known seal material may be used. The seal material includes a material that is not dissolved by the composition of the present disclosure, does not inhibit the polymerization reaction of the composition, and has sufficient heat resistance for the heating temperature during the heat treatment. Specific examples of a preferred seal material include a vinyl chloride resin gasket.

Next, the composition of the present disclosure is injected into a gap (void) defined by the cell prepared as described above by any suitable conventionally known method. Thereafter, the cell is subjected to heat treatment under the heating conditions described above. The method of the heat treatment for the cell into which the composition of the present disclosure is injected is not particularly limited. Similarly to the conventionally known cell cast method, the method of the heat treatment for the cell can be a method of directly performing heat treatment from the outside of the cell using, for example, a hot air circulation furnace or an infrared heater, and a method of further providing any suitable conventionally known jacket outside the cell and introducing a heat medium such as hot air, hot water, and water vapor into the jacket.

### <Applications of Polymethyl Methacrylate and Molded Body Thereof>

Polymethyl methacrylate obtained from the composition of the present disclosure and a molded body thereof have excellent light transmittance, heat resistance, and weather resistance. Therefore, the composition of the present disclosure can be suitably applied to various applications such as lighting equipment, automobile parts, signboards, and building materials that may be exposed to external environments, and also to heat sources and light sources.

### EXAMPLES

Hereinafter, embodiments of the present disclosure will be described based on Examples. The following Examples are not intended to limit the present disclosure.

### <Example 1>

A composition 1 was prepared by adding 0.005 mass% of toluene (manufactured by FUJIFILM Wako Pure Chemical Corporation) to 99.999 mass% of methyl methacrylate and mixing them. The resulting composition 1 was in a liquid state.

### <Examples 2 and 3 and Comparative Examples 1 and 2>

Compositions were prepared in the same manner as in Example 1, except that toluene added to methyl methacrylate and the concentration (mass ppm) thereof were changed as shown in Table 1 from Example 1. All of the resulting compositions were in a liquid state.

### (1) Evaluation of Storage Stability of Composition

The compositions prepared in Examples 1 to 3 and Comparative Examples 1 and 2 were subjected to a storage test including the following Steps 1 to 7 in this order. The storage test was carried out under accelerated conditions (80°C) in order to evaluate the stability after long-term storage.

Step 1: Inject 25 mL of the composition into a lower part of a pressure vessel ("TVS-N2 type" manufactured by Taiatsu Techno Corporation).

Step 2: A packing was interposed between the upper part and the lower part of the pressure vessel, and the pressure vessel was sealed.

Step 3: Nitrogen was introduced from the tip of the upper part of the pressure vessel, and sealed in a state where the internal pressure was 0.2 MPa, and it was confirmed whether the internal pressure did not change for 1 minute.

Step 4: The internal pressure in the pressure vessel was released, and a closing plug was attached to the tip of the upper part of the pressure vessel.

Step 5: The pressure vessel is placed in an oil bath set at 80°C.

Step 6: The pressure vessel is stored in the oil bath for 24 hours.

Step 7: After 24 hours had elapsed, the pressure vessel was taken out of the oil bath and placed in ice-cooled water for quenching.

The composition (3 mL) after the storage test was placed in a quartz glass cell (T-1, manufactured by DAICO MFG CO., Ltd.) with a size of 10 mm in length × 10 mm in width × 45 mm in height. Using the composition placed in the quartz glass cell, the average transmittance (%) was measured for light having a wavelength in a range of 300 nm to 370 nm when an optical path length was 10 mm. The average transmittance refers to an average value of transmittance values measured at every 5 nm in the wavelength range of 300 nm to 370 nm. The measurement was performed using a spectrophotometer ("Hitachi Spectrophotometer U-4000" manufactured by Hitachi High-Tech Fielding Corporation). As the average transmittance increases, it can be determined that optical properties such as high transparency are maintained even after storage. The results are shown in Table 1 and Fig. 1.

**[Table 1]**

| | Example 1 | Example 2 | Example 3 | Comparative Example 1 | Comparative Example 2 |
|---|---|---|---|---|---|
| Toluene [ppm] | 50 | 100 | 500 | 0 | 1000 |
| Average transmittance [%] | 98.23 | 99.00 | 98.13 | 98.05 | 96.90 |

As shown in Table 1 and Fig. 1, the compositions of Examples 1 to 3, in which toluene was added to methyl methacrylate at a concentration of less than 1,000 mass ppm, exhibited a higher average transmittance after the storage test than Comparative Example 1, in which toluene was not added to methyl methacrylate, and Comparative Example 2, in which toluene was added to methyl methacrylate at a concentration of 1,000 mass ppm.

The above results suggest that adding toluene to methyl methacrylate at a concentration of less than 1,000 mass ppm contributes to improving the storage stability of the composition.

## Claims

1. A composition comprising methyl methacrylate and toluene,
wherein a concentration of toluene is greater than 0 mass ppm and less than 1,000 mass ppm based on the total composition.

2. The composition according to claim 1, wherein a content of methyl methacrylate is 85 mass% or more based on the total composition.

3. The composition according to claim 1, wherein a content of methyl methacrylate is 90 mass% or more based on the total composition.

4. The composition according to claim 1, wherein the concentration of toluene is 5 mass ppm to 700 mass ppm based on the total composition.

5. The composition according to claim 1, wherein the concentration of toluene is 100 mass ppm to 600 mass ppm based on the total composition.

6. The composition according to claim 1, wherein the methyl methacrylate includes recycled methyl methacrylate or bio-derived methyl methacrylate.

7. The composition according to claim 1, further comprising a (meth)acrylic acid ester other than methyl methacrylate.

8. The composition according to claim 1, further comprising a polymer containing a structural unit derived from methyl methacrylate.

9. A polymer comprising a structural unit derived from the methyl methacrylate contained in the composition according to any one of claims 1 to 8.

10. A molded body comprising the polymer according to claim 9.

11. A cured product of the composition according to any one of claims 1 to 8.

12. A molded body comprising the cured product according to claim 11.

13. A method for producing polymethyl methacrylate, the method comprising polymerizing the methyl methacrylate contained in the composition according to any one of claims 1 to 8.
